# EUROPEAN PATENT APPLICATION

(11) **EP 4 342 458 A1**
(43) Date of publication of application: **27.03.2024**
(21) Application number: 22825365.4
(22) Date of filing: 17.06.2022
(51) Int. Cl.: A61K 9/20, A61K 31/4152

(54) **ORAL FORMULATION CONTAINING 1-(3-CYANO-1-ISOPROPYL-INDOL-5-YL)PYRAZOLE-4-CARBOXYLIC ACID**

(30) Priority: 17.06.2021 KR 20210078801
(71) Applicant: LG Chem, Ltd., Seoul 07336 (KR)
(72) Inventor: YOO, Seok Cheol, Daejeon 34122 (KR); JANG, Joomyung, Daejeon 34122 (KR); KIM, Ree Sun, Daejeon 34122 (KR); SEO, Jin A, Daejeon 34122 (KR)
(74) Representative: Hoffmann Eitle
(86) International application number: PCT/KR2022/008582
(87) International publication number: WO 2022/265442

(57) **Abstract**

The present invention relates to an oral formulation that contains an API selected from 1-(3-cyano-1-isopropyl-indol-5-yl)pyrazole-4-carboxylic acid or a pharmaceutically acceptable salt thereof, and does not contain pH adjusting agents as excipients. The oral formulation according to the present invention does not contain pH adjusting agents, and thus has the advantages of having increased productivity and convenience of dosage, as well as a high dissolution rate, despite having a high API content.

## Description

### [Technical Field]

The present invention relates to a composite formulation for oral dosage that comprises 1-(3-cyano-1-isopropyl-indol-5-yl)pyrazol-4-carboxylic acid) in a high content and has excellent average dissolution rate without comprising a pH modifier.

### [Background Art]

Xanthine oxidase is an enzyme that converts hypoxanthine to xanthine and also converts the formed xanthine to uric acid, and the substance inhibiting the activity of the xanthine oxidase may effectively treat diseases related to uric acid accumulation, such as hyperuricemia, gout, heart failure, and cardiovascular disease.

On the other hand, with respect to substances that inhibit the activity of xanthine oxidase, Korean Patent Publication No. 1751325 (Patent Document 1) provides 1-(3-cyano-1-isopropyl-indol-5-yl)pyrazol-4-carboxylic acid (Formula 1 below) and a method of preparing the compound; and Korean Patent Publication No. 1424013 (Patent Document 2) provides various types of crystal forms obtained using various solvents and a method of preparing the same.

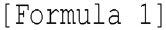

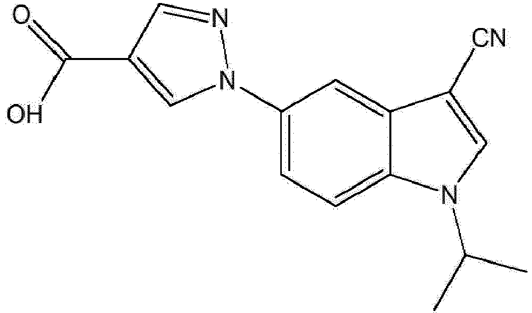

In the case of composite formulations for oral dosage, specifically, oral capsules or tablets, in order to increase pH-specific drug absorption, a method of inducing drug release at a specific pH by adding a pH modifier to increase solubility at a desired pH is generally used. In addition, apart from the use of the pH modifier, drug release is controlled by various methods that prevent absorption or release at a specific pH, such as enteric coating and enteric encapsulation that prevent dissolution or release by gastric acid before reaching the small intestine in order to induce absorption in the small intestine, which is the main absorption organ of the drug.

As such, when the pH modifier is included in the composite formulation for oral dosage or the enteric coating is added, an excipient or a coating layer is further included. Thus, in the case of comprising such a pH modifier or an enteric coating layer in a composite formulation for oral dosage comprising a high-dose active pharmaceutical ingredient (API), there is a problem that the size of the tablet or capsule may increase, thereby reducing patient dosing convenience as well as increasing the production cost.

In this regard, in the composite formulation for oral dosage comprising 1-(3-cyano-1-isopropyl-indol-5-yl)pyrazol-4-carboxylic acid of Formula 1 above or a pharmaceutically acceptable salt thereof as an active pharmaceutical ingredient (API) in a high content, there is a need for the development of a composite formulation for oral dosage that comprises a minimum of excipients and exhibits a high dissolution rate at pH 6.8, which is a desired dissolution pH, thereby having excellent patient dosing convenience and productivity.

### [Prior Art Documents]

### [Patent Documents]

(Patent Document 1) 1. Korean Patent Publication No. 1751325 (June 21, 2017) titled "novel compounds effective as xanthine oxidase inhibitors, method for preparing the same, and pharmaceutical composition containing the same"
(Patent Document 2) 2. Korean Patent Publication No. 1424013 (July 22, 2014) titled "1-(3-cyano-1-isopropyl-indol-5-yl)pyrazol-4-carboxylic acid crystalline form and the producing method thereof"

### [Disclosure]

### [Technical Problem]

It is an object of the present invention to derive a composite formulation for oral dosage having a specifically high dissolution rate at a desired pH of pH 6.8, while comprising a high content of 1-(3-cyano-1-isopropyl-indol-5-yl)pyrazol-4-carboxylic acid or a pharmaceutically acceptable salt thereof as an API.

### [Technical Solution]

The present invention provides a composite formulation for oral dosage that comprises 1-(3-cyano-1-isopropyl-indol-5-yl)pyrazol-4-carboxylic acid or a pharmaceutically acceptable salt thereof as an API and excipients, and in particular has a specifically high dissolution rate at a desired pH even without comprising pH modifier or an enteric coating layer among the excipients, and a method for preparing the composite formulation for oral dosage.

In the composite formulation for oral dosage of the present invention, when 900 ml of pH 6.8 buffer is used as a dissolution medium, and a paddle with a rotation speed of 50 rpm is used, a dissolution rate at 15 minutes is 65% or more, a dissolution rate at 30 minutes is 80% or more, and a dissolution rate at 60 minutes is 90% or more.

The API included in the composite formulation for oral dosage of the present invention has a content of 50 mg, 100 mg or 200 mg per the composite formulation.

The composite formulation for oral dosage of the present invention is used for the treatment or prevention of xanthine oxidase-related diseases selected from the group consisting of hyperuricemia, gout, heart failure, cardiovascular disease, hypertension, diabetes, kidney disease, inflammation and joint disease, and inflammatory bowel disease.

### [Advantageous Effects]

Since the composite formulation for oral dosage comprising 1-(3-cyano-1-isopropyl-indol-5-yl)pyrazol-4-carboxylic acid or a pharmaceutically acceptable salt thereof according to the present invention as an API has a dissolution property that shows a specifically high dissolution rate at a desired pH of pH 6.8 even without comprising a pH modifier or an enteric coating layer, high productivity through minimal use of additives and process simplification and patient dosing convenience due to small weight or size may be expected.

### [Description of Drawings]

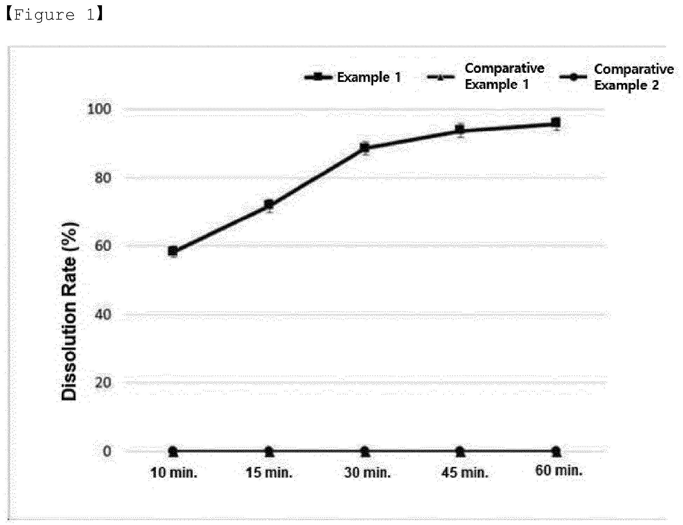

Figure 1 is a result of analyzing the average dissolution rate (%) of uncoated tablets according to the pH of a dissolution medium (buffer).

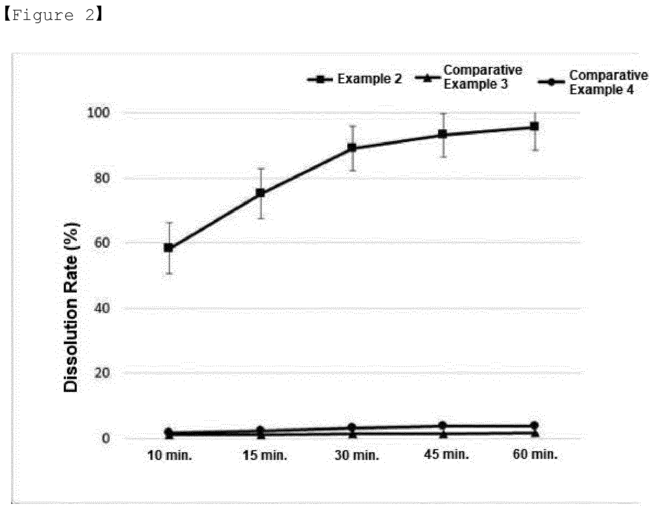

Figure 2 is a result of analyzing the average dissolution rate (%) of capsules according to the pH of a dissolution medium (buffer).

### [Best Mode]

Hereinafter, the present invention will be described in more detail.

Unless defined otherwise, all technical terms used in the present invention have the same meaning as commonly understood by those skilled in the art in the relevant field of the present invention. In addition, preferred methods or samples are described in the present specification, but similar or equivalent ones are included in the scope of the present invention. The contents of all publications described by reference in the present specification are incorporated in the present specification by reference in their entirety.

In the present invention, the terms "dissolution" and "release" refer to a phenomenon in which a composite formulation for oral dosage is dissolved in a buffer in the dissolution test method using the paddle method according to the dissolution test method in the Korean Pharmacopoeia, and the dissolution rate or average dissolution rate is confirmed by analyzing the concentration of the API dissolved in the buffer. In the present specification, the terms "dissolution" and "release" have the same meaning and may be used interchangeably without being distinguished from each other.

In the present invention, the term "composite formulation for oral dosage" may be any formulation known in the art, for example, tablets, capsules, granules, powders, oral solutions, syrups, or pills, but is not limited thereto. The composite formulation for oral dosage used in the examples of the present invention is a tablet or capsule comprising an API of 1-(3-cyano-1-isopropyl-indol-5-yl)pyrazol-4-carboxylic acid and pharmaceutically acceptable additives.

The term "pharmaceutically acceptable salt" in the present invention refers to a salt form of a compound that does not cause serious irritation to the organism to which the compound is administered and does not impair the biological activity and physical properties of the compound. 1-(3-Cyano-1-isopropyl-indol-5-yl)pyrazol-4-carboxylic acid, which is an API included in the composite formulation for oral dosage of the present invention, may be converted into a salt thereof by a conventional method.

The term "human xanthine oxidase-related disease" of the present invention refers to a disease that may be treated or prevented by inhibiting human xanthine oxidase, and include, for example, hyperuricemia, gout, heart failure, cardiovascular disease, hypertension, diabetes, diabetes-related complications, kidney disease, joint disease, inflammatory bowel disease, and the like, but is not limited to the above-mentioned diseases. Examples of the diabetes-related complications include hyperlipidemia, arteriosclerosis, obesity, hypertension, retinopathy, renal failure, and the like.

The term "treating" means stopping or delaying the progression of a disease when used in a subject showing the onset of symptoms, and the term "preventing" means stopping or delaying the signs of the onset of symptoms when used in a subject who does not show the onset of symptoms but is at high risk thereof.

In the present invention, various studies were conducted on a composite formulation for oral dosage having excellent or good bioavailability represented by a dissolution rate while comprising an API selected from 1-(3-cyano-1-isopropyl-indol-5-yl)pyrazol-4-carboxylic acid or a pharmaceutically acceptable salt thereof in a high content.

As a result of continuous research in various ways to increase the content of API and to maintain or increase the physical properties in the composite formulation for oral dosage comprising 1-(3-cyano-1-isopropyl-indol-5-yl)pyrazol-4-carboxylic acid or a pharmaceutically acceptable salt thereof as the API, the present inventors have developed a composite formulation for oral dosage that may be dissolved at a specifically high level at a desired pH of pH 6.8 even without comprising a pH modifier or with uncoated tablets and capsules that do not comprise an additional enteric coating.

In this case, since it does not comprise a pH modifier or an enteric coating layer as an excipient that may be included in the composite formulation for oral dosage, there is an advantage that the dosing convenience and productivity may be increased by comprising a minimized content of the excipient.

Therefore, the present invention provides a composite formulation for oral dosage having a high dissolution rate, which comprises 1-(3-cyano-1-isopropyl-indol-5-yl)pyrazol-4-carboxylic acid or a pharmaceutically acceptable salt thereof and does not comprise a pH modifier or an enteric coating layer as an excipient. The composite formulation for oral dosage may be a tablet or capsule.

In preparing a composite formulation for oral dosage, various types of pharmaceutically acceptable excipients are added to optimize basic physical properties such as tabletting property, hardness, friability, and flowability. In addition to this, the composite formulation for oral dosage must first pass through the digestive system including the stomach for its action, and the stomach may cause degradation of APIs in a strong acidic environment of pH 1 to 2, so that various release control methods are being studied to prevent dissolution (or release) of the composite formulation for oral dosage in an acidic environment.

A representative method among the release control methods of the composite formulation for oral dosage includes comprising an enteric coating layer on the outside of the composite formulation for oral dosage to prevent dissolution in an acidic environment, or using a pH modifier for dissolution in a desired pH environment, or using sustained-release technology using various types of polymers. However, all of these methods have disadvantages in that the use of additional excipients or the addition of a coating layer reduces dosing convenience due to increase in the size of the formulation, as well as lowering productivity due to an additional process in the production process.

The composite formulation for oral dosage of the present invention is a composite formulation for oral dosage having excellent dissolution rate even without comprising a pH modifier or an enteric coating layer.

The present invention provides a composite formulation for oral dosage that i) comprises 1-(3-cyano-1-isopropyl-indol-5-yl)pyrazol-4-carboxylic acid or a pharmaceutically acceptable salt thereof as an API and ii) does not comprise a pH modifier or an enteric coating layer, and a method of preparing the composite formulation for oral dosage.

Since the composite formulation for oral dosage of the present invention in an uncoated state was hardly dissolved when a pH 1.2 buffer or a pH 4.5 buffer was used as a dissolution medium, the composite formulation for oral dosage of the present invention does not require an enteric coating layer. In addition, the composite formulation for oral dosage of the present invention has a very low dissolution rate at 60 minutes of less than 10% when purified water is used as a dissolution medium.

In the composite formulation for oral dosage of the present invention, when 900 ml of pH 6.8 buffer is used as a dissolution medium, and a paddle with a rotation speed of 50 rpm is used, a dissolution rate at 15 minutes in an uncoated tablet is 65% or more, a dissolution rate at 30 minutes is 80% or more, and a dissolution rate at 60 minutes is 90% or more. Thus, since the composite formulation for oral dosage of the present invention exhibits the effect of having a specifically very excellent dissolution rate at a desired pH of pH 6.8 even without comprising a pH modifier, its bioavailability may also be expected to be excellent by reflecting this.

Taking these results together, it may be predicted that the composite formulation for oral dosage of the present invention is hardly dissolved in the stomach, but is specifically dissolved in the intestine and absorbed in the body.

In the present invention, the composite formulation for oral dosage further comprises one or more excipients selected from pharmaceutically acceptable diluents, disintegrants, glidants, lubricants, and the like, as excipients.

As excipients such as the diluents, disintegrants, glidants, and lubricants, any of those known to be commonly used in the art may be used. The diluent may be used in an amount of 30 to 50% by weight, 40 to 50% by weight, or 45 to 50% by weight based on the total weight of the composite formulation for oral dosage. The disintegrant may be used in an amount ranging from 1 to 10% by weight or from 1 to 5% by weight based on the total weight of the composite formulation for oral dosage. The glidant may be used in an amount ranging from 0.1 to 5% by weight, 0.2 to 3% by weight, or 0.3 to 2% by weight based on the total weight of the oral tablet. The lubricant may be used in an amount ranging from 0.1 to 10% by weight, 0.3 to 5% by weight, or 0.5 to 4% by weight based on the total weight of the composite formulation for oral dosage.

For example, the diluent may be selected from the group consisting of, but is not limited to, microcrystalline cellulose (MCC), lactose monohydrate, lactose anhydride, lactose, starch, mannitol, carboxymethylcellulose, sorbitol, and combinations thereof. The disintegrant may be selected from the group consisting of, but is not limited to, low-substituted hydroxypropyl cellulose, crospovidone, croscarmellose sodium, sodium starch glycolate, F-melt, and combinations thereof. The glidant may be selected from the group consisting of, but is not limited to, talc, silicon dioxide, and mixtures thereof. The lubricant may be selected from the group consisting of, but is not limited to, magnesium stearate, silicon dioxide, talc, light anhydrous silicic acid, sodium stearyl fumarate, and combinations thereof.

The composite formulation for oral dosage may be administered once a day, and may be taken daily.

The content of the API included in the composite formulation for oral dosage is 30 to 50% by weight, 35 to 50% by weight, 40 to 50% by weight, 45 to 50% by weight, 30 to 45% by weight, 35 to 45% by weight, 40 to 45% by weight, 30 to 40% by weight, or 35 to 40% by weight, based on the total weight of the composite formulation for oral dosage.

The API may be included in an amount of 50 to 500 mg, 50 to 400 mg, 50 to 300 mg, 50 to 200 mg, 50 to 100 mg, 100 to 500 mg, 100 to 400 mg, 100 to 300 mg, 100 to 200 mg, 200 to 500 mg, 200 to 400 mg, 200 to 300 mg, 300 to 500 mg, or 300 to 400 mg per unit dosage form.

The API may be included in an amount of, for example, 50 mg, 100 mg, 150 mg, 200 mg, 300 mg, 400 mg, or 455 mg per unit dosage form.

The content of the API included in the composite formulation for oral dosage of the present invention may be 95% to 105%, 96% to 105%, 97% to 105%, 98% to 105%, 99% to 105%, 100% to 105%, 95% to 100 %, 96% to 100%, 97% to 100%, 98% to 100%, or 99% to 100%. In addition, the API content may be 95% or more, 96% or more, 97% or more, 98% or more, or 99% or more, and may be 105% or less, 104% or less, 103% or less, 102% or less, 101% or less, or 100% or less.

The present invention provides a composite formulation for oral dosage for treating or preventing human xanthine oxidase-related diseases, which comprises the 1-(3-cyano-1-isopropyl-indol-5-yl)pyrazol-4-carboxylic acid or a pharmaceutically acceptable salt thereof as an API and does not comprise a pH modifier or an enteric coating layer as an excipient.

The present invention provides a method for treating or preventing human xanthine oxidase-related diseases using the composite formulation for oral dosage that comprises the 1-(3-cyano-1-isopropyl-indol-5-yl)pyrazol-4-carboxylic acid or a pharmaceutically acceptable salt thereof as an API and does not comprise a pH modifier or an enteric coating layer as an excipient.

The present invention provides the use of a composite formulation for oral dosage for treating or preventing human xanthine oxidase-related diseases, which comprises the 1-(3-cyano-1-isopropyl-indol-5-yl)pyrazol-4-carboxylic acid or a pharmaceutically acceptable salt thereof as an API and does not comprise a pH modifier or an enteric coating layer as an excipient.

Unless otherwise indicated, all numbers used in the specification and claims, whether recited or not, are to be understood as being modifiable by the term "about" in all instances. It is also to be understood that the precise numbers used in the specification and claims form additional embodiments of the present disclosure. Efforts have been made to ensure the accuracy of the numerical values disclosed in the examples. However, all measured values may inherently include certain error values generated from the standard deviations measured in their respective measurement techniques.

### [Test Examples and Examples]

Various evaluations in the examples and the comparative examples were performed as follows.

The oral tablets of the examples and the comparative examples were prepared as uncoated tablets using the ingredients in the corresponding contents as shown in Table 1 below (Example 1, and Comparative Examples 1 and 2), and the oral capsules were prepared as capsules using the ingredients in the corresponding contents as shown in Table 2 below (Example 2, and Comparative Examples 3 and 4).

Oral tablets (uncoated tablets) were prepared by the following preparation method.

1-(3-cyano-1-isopropyl-indol-5-yl)pyrazol-4-carboxylic acid (API) and a glidant were mixed and then milled to prepare a first mixture. Thereafter, a diluent, a disintegrant and a lubricant, which are ingredients not included in the first mixture, were mixed, and then milled to prepare a second mixture.

After the first mixture and the second mixture were mixed, tableting was performed under conditions of prepressure of 5.0 kN and main pressure of 14 to 15 kN using a rotary tableting machine (Modul P, GEA, Belgium) to prepare uncoated tablets.

PRUV^{®} used as a lubricant is a trade name, and its ingredient is sodium stearyl fumarate.

Oral capsules were prepared by the following preparation method.

1-(3-Cyano-1-isopropyl-indol-5-yl)pyrazol-4-carboxylic acid (API) and a diluent were mixed and then milled to prepare a first mixture. Thereafter, a lubricant, which is an ingredient not included in the first mixture, was mixed, and then milled to prepare a second mixture.

The second mixture was prepared by putting 430.0 mg into a capsule using a capsule filling machine (manual or automatic).

**[Table 1]**

| Classification | Ingredient | Content (mg/T) | Content Ratio (%) |
|---|---|---|---|
| API | 1-(3-Cyano-1-isopropyl-indol-5-yl)pyrazol-4-carboxylic acid | 100.0 | 45.5 |
| Diluent | MCC 102 (Microcrystalline cellulose) | 104.8 | 47.9 |
| Disintegrant | Crospovidone | 9.7 | 4.4 |
| Glidant | Colloidal silicon dioxide (SiO₂) | 1.1 | 0.2 |
| Lubricant | PRUV^{®} | 4.4 | 2.0 |
| Tablet total weight (mg) | | 220.0 | 100.0 |

**[Table 2]**

| Classification | Ingredient | Content (mg/T) | Content Ratio (%) |
|---|---|---|---|
| API | 1-(3-Cyano-1-isopropyl-indol-5-yl)pyrazol-4-carboxylic acid | 100.0 | 23.3 |
| Diluent | Starcap 1500^{®} | 326.0 | 75.8 |
| Lubricant | Magnesium stearate | 4.0 | 0.9 |
| Total weight of mixture (mg) | | 430.0 | 81.59 |
| Capsule | Hard gelatin capsule | 97.0 | 18.41 |
| Total weight of capsule (mg) | | 527.0 | 100.0 |

(Since the weight of the capsules varies between batches, it was selected based on 97.0 mg, which is the average of the capsule weight range of a maximum of 106.7 mg and a minimum of 87.3 mg.)

### [Dissolution rate analysis]

The previously prepared uncoated tablets were tested for dissolution using the dissolution test solution (dissolution medium) of the following examples and comparative examples according to the dissolution testing method in the Korean Pharmacopoeia (Tenth Edition). The dissolution method was performed using a paddle method according to the dissolution testing method in the Korean Pharmacopoeia with a stirring speed of 50 rpm and a dissolution temperature of 37±0.5°C.

In Example 1, the dissolution rate of the previously prepared uncoated tablet was measured in 900 ml of a pH 6.8 phosphate buffer/water mixture (1:1), which is a second solution of the dissolution test method in the Korean Pharmacopoeia.

In Comparative Example 1, 1000 ml of a first solution was prepared by dissolving 2.0 g of sodium chloride in 7.0 ml of hydrochloric acid and water, which is a first solution of dissolution test method in the Korean Pharmacopoeia, and then the dissolution rate of the previously prepared uncoated tablet was measured using 900 ml of the first solution. The first solution has a pH of 1.2, and hydrochloric acid has a concentration of 0.1 mol/L and is colorless and transparent.

In Comparative Example 2, 1000 ml of a buffer at a pH of 4.5was prepared by dissolving 2.99 g of sodium acetate hydrate (trihydrate) and 1.66 g of acetic anhydride in water. The dissolution rate of the previously prepared uncoated tablets was measured using 900 ml of the pH 4.5 buffer as a dissolution medium.

In analytical conditions, the solution obtained in the dissolution test was filtered through a 0.45 um membrane filter, and the concentration of 1-(3-cyano-1-isopropyl-indol-5-yl)pyrazol-4-carboxylic acid as API was analyzed using the UPLC method. Specific analytical conditions are as follows.

In Example 2, the dissolution rate of the previously prepared capsule was measured in the same dissolution medium and conditions as in Example 1.

In Comparative Examples 3 and 4, the dissolution rate of the previously prepared capsules was measured in the same dissolution medium and conditions as in Comparative Examples 1 and 2, respectively.

### <Analytical Conditions>

Preparation of mobile phase: acetonitrile (500 ml) + purified water (500 ml) + TFA (1 ml)
Preparation of diluent: methanol (900 ml) + purified water (100 ml)
Preparation of standard solution and test solution: After the standard and sample are completely dissolved in the diluent, the analysis is performed according to the UPLC analytical method below.
Column: Waters CSH C18 (2.1 mm I.D. X 100 mm L, Particle size 1.7 um)
Column temperature: 40°C
Mobile phase: acetonitrile/H₂O/TFA = 500/500/1 (v/v/v)
Flow rate: 0.35 ml/min
Detection: 258 nm uv
Sample amount: 1 µl
Analytical time: 6 minutes

### Dissolution patterns in examples and comparative examples

In the oral tablet (uncoated tablet) comprising 1-(3-cyano-1-isopropyl-indol-5-yl)pyrazol-4-carboxylic acid as an API, the dissolution patterns in the dissolution solvents of pH 6.8 (Example 1), pH 1.2 (Comparative Example 1) and pH 4.5 (Comparative Example 2) were analyzed through the aforementioned analytical method according to the dissolution test method in the Korean Pharmacopoeia. The analytical results are shown in Table 3.

**[Table 3]**

| | Example 1 (pH 6.8) | Comparative Example 1 (pH 1.2) | Comparative Example 2 (pH 4.5) |
|---|---|---|---|
| Average Dissolution Rate (10 minutes) (%) | 58.4±1.6 | N/D | N/D |
| Average dissolution rate (15 minutes) (%) | 71.8±1.8 | N/D | N/D |
| Average dissolution rate (30 minutes) (%) | 88.6±1.9 | N/D | N/D |
| Average Dissolution Rate (45 minutes) (%) | 93.8±2.0 | N/D | N/D |
| Average Dissolution Rate (60 minutes) (%) | 95.8±2.0 | N/D | N/D |

| | | | |
|---|---|---|---|
| (N/D: Not detected) | | | |

The tablets (uncoated tablets) prepared by using the ingredients in the corresponding contents as shown in Table 1 were hardly dissolved in buffers of pH 1.2 (Comparative Example 1) and pH 4.5 (Comparative Example 2). However, in a buffer of pH 6.8, the average dissolution rate at 10 minutes was 58.4±1.6%, the average dissolution rate at 15 minutes was 71.8±1.8%, the average dissolution rate at 30 minutes was 88.6±1.9%, the average dissolution rate at 45 minutes was 93.8±2.0%, and the average dissolution rate at 60 minutes was 95.8±2.0%.

The dissolution patterns of the oral capsule comprising the same API as the uncoated tablet in the buffers of pH 6.8 (Example 2), pH 1.2 (Comparative Example 3) and pH 4.5 (Comparative Example 4) were analyzed through the aforementioned analytical method according to the dissolution test method in the Korean Pharmacopoeia. The analytical results are shown in Table 4.

**[Table 4]**

| | Example 2 (pH 6.8) | Comparative Example 3 (pH 1.2) | Comparative Example 4 (pH 4.5) |
|---|---|---|---|
| Average Dissolution Rate (10 minutes) (%) | 58.4±7.9 | 1.2±0.0 | 1.9±0.2 |
| Average Dissolution Rate (15 minutes) (%) | 75.2±7.8 | 1.3±0.0 | 2.3±0.2 |
| Average Dissolution Rate (30 minutes) (%) | 89.1±6.9 | 1.5±0.0 | 3.2±0.2 |
| Average Dissolution Rate (45 minutes) (%) | 93.1±6.6 | 1.6±0.0 | 3.7±0.1 |
| Average Dissolution Rate (60 minutes) (%) | 95.5±6.9 | 1.7±0.1 | 3.9±0.1 |

The oral capsules prepared by using the ingredients in the corresponding contents as shown in Table 2 were hardly dissolved in the buffers of pH 1.2 (Comparative Example 2) and pH 4.5 (Comparative Example 3), like the tablets (uncoated tablets), and in a buffer of pH 6.8, the average dissolution rate at 10 minutes was 58.4±7.9%, the average dissolution rate at 15 minutes was 75.2±7.8%, the average dissolution rate at 30 minutes was 89.1±6.9%, the average dissolution rate at 45 minutes was 93.1±6.6%, and the average dissolution rate at 60 minutes was 95.5±6.9%, and thus, it was confirmed that tablets and capsules comprising the 1-(3-cyano-1-isopropyl-indol-5-yl)pyrazol-4-carboxylic acid as an API showed a similar dissolution pattern.

Thus, since the composite formulation for oral dosage of the present invention is hardly dissolved at pH 1.2 even in an uncoated state, there is an advantage in that it is not necessary to apply an enteric coating to prevent dissolution in the stomach when the composite formulation for oral dosage is in the form of a tablet. In addition, since the composite formulation for oral dosage of the present invention exhibits a very high dissolution rate with an average dissolution rate specifically at 60 minutes of 90% or more at pH 6.8 even without comprising a pH modifier, regardless of tablet or capsule formulations, its bioavailability may also be expected to be excellent by reflecting this.

So far, the present invention has been described with reference to the preferred embodiments. However, it will be understood by those of ordinary skill in the art to which the present invention pertains that the present invention may be implemented in a modified form without departing from the essential characteristics of the present invention. Therefore, the disclosed embodiments should be considered as being exemplary and not limiting. The scope of the present invention is defined in the claims rather than the detailed description, and all differences within its equivalent range should be interpreted as being included in the invention.

## Claims

1. A composite formulation for oral dosage comprising an active pharmaceutical ingredient (API) selected from 1-(3-cyano-1-isopropyl-indol-5-yl)pyrazol-4-carboxylic acid or a pharmaceutically acceptable salt thereof, wherein the composite formulation for oral dosage does not comprise a pH modifier as an excipient.

2. The composite formulation for oral dosage according to claim 1, wherein in the composite formulation for oral dosage, when 900 ml of pH 6.8 buffer is used as a dissolution medium, and a paddle with a rotation speed of 50 rpm is used, a dissolution rate at 15 minutes is 65% or more, a dissolution rate at 30 minutes is 80% or more, a the dissolution rate at 60 minutes is 90% or more.

3. The composite formulation for oral dosage according to claim 2, wherein in the composite formulation for oral dosage, when a pH 1.2 buffer or a pH 4.5 buffer is used as a dissolution medium, and a paddle with a rotation speed of 50 rpm is used, a dissolution at 60 minutes is not detected.

4. The composite formulation for oral dosage according to any one of claims 1 to 3, wherein the composite formulation for oral dosage is an oral tablet or an oral capsule.

5. The composite formulation for oral dosage according to claims 4, wherein the composite formulation for oral dosage is an oral tablet.

6. The composite formulation for oral dosage according to claim 5, wherein the oral tablet does not further comprise an enteric coating layer.

7. The composite formulation for oral dosage according to claim 6, wherein the API has a content of 30 to 55% by weight based on the total weight of the composite formulation.

8. The composite formulation for oral dosage according to claim 7, wherein the API has a content of 40 to 50% by weight based on the total weight of the composite formulation.

9. The composite formulation for oral dosage according to claim 1, wherein the API has a content of 50 mg, 100 mg, 200 mg or 300 mg per unit dosage form.
